(19) Europäisches Patentamt
European Patent Office
Office européen des brevets

(11) Veröffentlichungsnummer: **0 073 912**
**B1**

(12) # EUROPÄISCHE PATENTSCHRIFT

(45) Veröffentlichungstag der Patentschrift: **20.03.85**

(21) Anmeldenummer: **82106478.9**

(22) Anmeldetag: **19.07.82**

(51) Int. Cl.⁴: **C 07 F 9/65**, C 07 D 239/95, A 01 N 57/16, A 01 N 57/24, A 01 N 57/32

(54) **Oxo-chinazolin-(thiono)-phosphor(phosphon)-säureester bzw.esteramide, Verfahren zu ihrer Herstellung und ihre Verwendung als Schädlingsbekämpfungsmittel.**

(30) Priorität: **31.07.81 DE 3130344**

(43) Veröffentlichungstag der Anmeldung: **16.03.83 Patentblatt 83/11**

(45) Bekanntmachung des Hinweises auf die Patenterteilung: **20.03.85 Patentblatt 85/12**

(84) Benannte Vertragsstaaten: **BE CH DE FR GB IT LI NL**

(56) Entgegenhaltungen:
**DE - A - 2 223 025**

**DERWENT JAPANESE PATENTS, Band 8, Nr. 16, Woche 17-04-69 - 23-04-69, Teil 5, Seite 4**
**DERWENT JAPANESE PATENTS, Band 8, Nr. 2, Woche 09-01-69 - 14-01-69, Teil 5, Seite 1**
**Journal of Heterocyclic Chemistry, 1972, vol. 9, pages 569-579**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**

**EP 0 073 912 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Oxochinazolin(thiono)phosphor(phosphon)säureester bzw. -esteramide, ein Verfahren zu ihrer Herstellung und ihre Verwendung als Schädlingsbekämpfungsmittel, insbesondere als Insektizide und Akarizide.

Es ist bekannt, dass bestimmte Oxochinazolinthionophosphorsäureester, wie z.B. O,O-Diethyl-O-3,4-dihydro-4-oxochinazolin-3-ylthionophosphorsäureester und O,O-Diethyl-O-3,4-dihydro-2-methyl-4-oxochinazolin-3-ylthionophosphorsäureester zur Schädlingsbekämpfung verwendet werden können (vgl. DE-OS Nr. 2223025 und JP Nr. 8508/69). Die insektizide und akarizide Wirkung der bekannten Verbindungen ist jedoch, insbesondere bei niedrigen Wirkstoffkonzentrationen und Aufwandmengen, nicht immer zufriedenstellend.

Es wurden neue Oxochinazolin(thiono)phosphor(phosphon)säureester bzw. -esteramide der Formel I

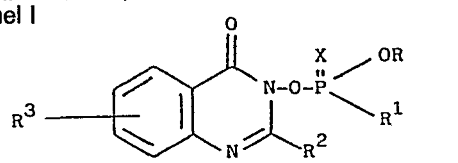

(I)

gefunden, in welcher

R für gegebenenfalls substituiertes Alkyl steht,

$R^1$ für einen gegebenenfalls substituierten Alkyl-, Alkoxy-, Alkylthio-, Alkylamino-, Dialkylamino- oder Arylrest steht,

$R^2$ für einen gegebenenfalls substituierten Alkoxy-, Alkylthio-, Alkylamino- oder Dialkylaminorest steht,

$R^3$ für Wasserstoff oder Halogen steht, und

X für Sauerstoff oder Schwefel steht.

Man erhält die neuen Verbindungen der Formel I, wenn man 3,4-Dihydro-3-hydroxy-4-oxochinazoline der Formel II

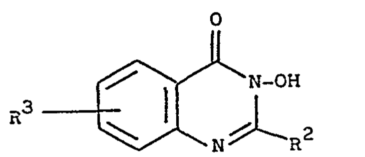

(II)

in welcher

$R^2$ und $R^3$ die oben angegebene Bedeutung haben,

mit (Thiono)Phosphor(phosphon)säureester- bzw. -esteramidhalogeniden der Formel III

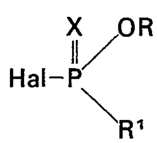

(III)

in welcher

R, $R^1$ und X die oben angegebene Bedeutung haben, und

Hal für Halogen (vorzugsweise Chlor) steht,

gegebenenfalls in Gegenwart von Säureakzeptoren und gegebenenfalls in Gegenwart von Verdünnungsmitteln umsetzt.

Die neuen Oxochinazolin(thiono)phosphor(phosphon)säureester bzw. -esteramide der Formel I zeichnen sich durch hohe Wirksamkeit gegen tierische Schädlinge, insbesondere durch hohe insektizide und akarizide Wirksamkeit aus.

Überraschenderweise zeigen die erfindungsgemässen Verbindungen der Formel I erheblich höhere insektizide und akarizide Wirkung als vergleichbare bekannte Verbindungen.

Die Erfindung betrifft insbesondere Verbindungen der Formel I, in welcher

R für gegebenenfalls substituiertes Alkyl mit 1 bis 6 Kohlenstoffatomen steht,

$R^1$ für einen gegebenenfalls substituierten Alkyl-, Alkoxy-, Alkylthio-, Alkylamino- oder Dialkylaminorest mit jeweils 1 bis 6 Kohlenstoffatomen je Alkylteil oder Phenyl steht,

$R^2$ für einen gegebenenfalls substituierten Alkoxy-, Alkylthio-, Alkylamino- oder Dialkylaminorest mit jeweils 1 bis 6 Kohlenstoffatomen je Alkylteil steht,

$R^3$ für Wasserstoff, Fluor, Chlor oder Brom steht, und

X für Sauerstoff oder Schwefel steht.

Alkyl R und $R^1$, Phenyl $R^1$ sowie Alkoxy, Alkylthio und (Di)Alkylamino $R^1$ und $R^2$ können einen oder mehrere, vorzugsweise 1 bis 3, insbesondere 1 oder 2 gleiche oder verschiedene Substituenten tragen. Beispielsweise seien genannt: Halogene, wie Fluor, Chlor und Brom, Cyano sowie Alkoxy und Alkylthio mit 1 bis 5, insbesondere 1 bis 3 Kohlenstoffatomen.

Bevorzugt sind die Reste R, $R^1$ und $R^2$ unsubstituiert oder der Alkoxyrest $R^1$ ist durch Alkoxy, insbesondere Methoxy oder Ethoxy substituiert.

Die Alkylreste R und $R^1$ sowie die Alkylteile der Alkoxyreste $R^1$ und $R^2$ und der Alkylthio-, Alkylamino- und Dialkylaminoreste $R^1$ und $R^2$ können verzweigt oder unverzweigt sein und jeweils 1 bis 6, insbesondere 1 bis 4 Kohlenstoffatome enthalten.

Besonders bevorzugt steht R für Methyl, Ethyl, n-Propyl, iso-Propyl, n-Butyl, iso-Butyl und tert.-Butyl; $R^1$ für Methyl, Ethyl, n-Propyl, iso-Propyl, n-Butyl, iso-Butyl, tert.-Butyl und Phenyl; $R^1$ und $R^2$ für Methoxy, Ethoxy, Methoxymethoxy, Methoxyethoxy, n-Propoxy, iso-Propoxy, n-Butoxy, sek.-Butoxy, iso-Butoxy, tert.-Butoxy, Methylthio, Ethylthio, n-Propylthio, iso-Propylthio, n-Butylthio, iso-Butylthio, sek.-Butylthio, tert.-Butylthio, (Di)Methylamino, (Di)Ethylamino, (Di)-n-Propylamino, (Di)-iso-Propylamino, (Di)-n-Butylamino, (Di)-iso-Butylamino, (Di)-sek.-Butylamino und (Di)-tert.-Butylamino; $R^3$ für Wasserstoff, Chlor und Brom und X für Sauerstoff oder Schwefel.

Verwendet man beispielsweise 7-Chlor-3,4-dihydro-2-ethoxy-3-hydroxy-4-oxochinazolin und O-Ethylethanphosphorsäureesterchlorid als Ausgangsstoffe, so kann die Reaktion dieser Verbindungen durch folgendes Formelschema skizziert werden

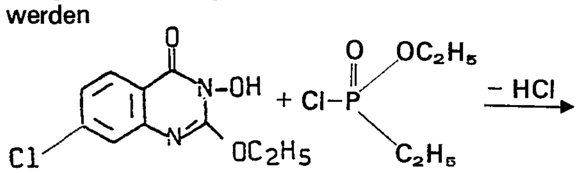

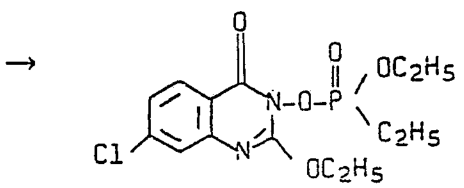

Die beim erfindungsgemässen Verfahren als Ausgangsstoffe zu verwendenden neuen 3,4-Dihydro-3-hydroxy-4-oxochinazoline sind durch die Formel II definiert. In dieser Formel haben die Reste $R^2$ und $R^3$ die oben bei Formel I angegebene Bedeutung.

Als Beispiele für die Verbindungen der Formel II seien genannt:

2-Methoxy-, 2-Ethoxy-, 2-n-Propoxy-, 2-iso-Propoxy-, 2-n-Butoxy-, 2-sek.-Butoxy-, 2-iso-Butoxy-, 2-tert.-Butoxy-, 2-Methylthio-, 2-Ethylthio-, 2-n-Propylthio-, 2-iso-Propylthio-, 2-n-Butylthio-, 2-sek.-Butylthio-, 2-iso-Butylthio-, 2-tert.-Butylthio-, 2-(Di)Methylamino-, 2-(Di)-Ethylamino-, 2-(Di)-n-Propylamino-, 2-(Di)-iso-Propylamino-, 2-(Di)-n-Butylamino-, 2-(Di)-iso-Butylamino-, 2-(Di)-sek.-Butylamino-, 2-(Di)-tert.-Butylamino-3,4-dihydro-3-hydroxy-4-oxochinazoline bzw. -7-chlor(brom)-3,4-dihydro-3-hydroxy-4-oxochinazoline, -6-chlor-(brom)-3,4-dihydro-3-hydroxy-4-oxochinazolin.

Die Verbindungen der Formel II sind neu. Die Herstellung geschieht nach allgemein üblichen Methoden, indem man z.B. 2-Aminohydroxamsäuren der Formel IV

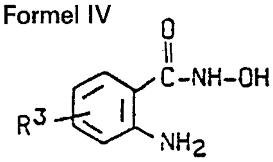 (IV)

in welcher

$R^3$ die oben angegebene Bedeutung hat,

mit Orthoestern der Formel V

$$R^2-C(OR^4)_3 \qquad (V)$$

in welcher

$R^2$ die oben angegebene Bedeutung hat, und

$R^4$ für Methyl oder Ethyl steht,

gegebenenfalls in Gegenwart von Verdünnungsmitteln wie z.B. Methanol, bei Temperaturen zwischen 20 und 160°C umsetzt (vgl. DE-OS Nr. 2223025).

Die als Ausgangsstoffe benötigten O-Aminohydroxamsäuren der Formel IV sind allgemein bekannte Verbindungen der organischen Chemie. Als Beispiele seien genannt:

2-Amino-, 2-Amino-4-chlor-, 2-Amino-4-brom-, 2-Amino-5-chlor-, 2-Amino-5-brombenzhydroxamsäure.

Die ausserdem als Ausgangsstoffe benötigten Orthoester der Formel V sind allgemein bekannte Verbindungen der organischen Chemie. Als Beispiele seien genannt:

O-Methyl-, O-Ethyl-, O-n-Propyl-, O-iso-Propyl-, O-n-Butyl-, O-iso-Butyl-, O-sek.-Butyl-, O-tert.-Butyl-O,O,O-trimethylorthokohlensäureester bzw. -O,O,O-triethylorthokohlensäureester; S-Methyl-, S-Ethyl-, S-n-Propyl-, S-iso-Propyl-, S-n-Butyl-, S-iso-Butyl-, S-sek.-Butyl-, S-tert.-Butyl-O,O,O-trimethylorthothiokohlensäureester bzw. -O,O,O-triethylorthothiokohlensäureester; O,O,O,N-Orthokohlensäuretetramethylesteramid, O,O,O,N,N-Orthokohlensäurepentamethylesteramid; O,O,O,N-Orthokohlensäuretetraethylesteramid; O,O,O,N,N,-Orthokohlensäurepentaethylesteramid; N-n-Propyl-N,N-Di-n-Propyl-, N-iso-Propyl-, N,N-Di-iso-Propyl-, N-n-Butyl-, N,N-Di-n-Butyl-, N-iso-Butyl-, N,N-Di-iso-Butyl-, N-sek.-Butyl-, N-tert.-Butyl-O,O,O-trimethylorthokohlensäureesteramid bzw. -O,O,O-triethylorthokohlensäureesteramid.

Die weiterhin für das erfindungsgemässe Verfahren als Ausgangsstoffe zu verwendende (Thiono)Phosphor(phosphon)säureester- bzw. -esteramidhalogenide sind durch die Formel III definiert. In dieser Formel haben die Reste R und $R^1$ die bei Formel I angegebene Bedeutung. Hal steht für Halogen, vorzugsweise für Chlor.

Als Beispiele für die Verbindungen der Formel III seien genannt:

O-Methyl-, O-Ethyl-, O-n-Propyl-, O-iso-Propyl-, O-n-Butyl-, O-iso-Butyl- und O-sek.-Butyl-(thiono)methanphosphonsäureesterchlorid;

O-Methyl-, O-Ethyl, O-n-Propyl-, O-iso-Propyl-, O-n-Butyl-, O-iso-Butyl- und O-sek.-Butyl(thiono)ethanphosphonsäureesterchlorid;

O-Methyl-, O-Ethyl-, O-n-Propyl-, O-iso-Propyl-, O-n-Butyl-, O-iso-Butyl- und O-sek.-Butyl-(thiono)propanphosphonsäureesterchlorid;

O-Methyl-, O-Ethyl-, O-n-Propyl-, O-iso-Propyl-, O-n-Butyl-, O-iso-Butyl- und O-sek.-Butyl-(thiono)butanphosphonsäureesterchlorid;

O,O-Dimethyl-, O,O-Diethyl-, O,O-Di-n-propyl-, O,O-Di-n-butyl-, O-Methyl-O-ethyl-, O-Methyl-O-n-propyl-, O-Methyl-O-iso-propyl-, O-Methyl-O-n-butyl-, O-Methyl-O-iso-butyl-, O-Ethyl-O-n-propyl-, O-Ethyl-O-n-butyl-, O-Ethyl-O-iso-butyl-, O-n-Propyl-O-n-butyl-, und O-n-Propyl-O-isobutyl(thiono)phosphorsäurediesterchlorid;

O,S-Dimethyl-, O,S-Diethyl-, O,S-Di-n-propyl-, O,S-Di-iso-propyl-, O-Methyl-S-ethyl-, O-Methyl-S-n-propyl-, O-Methyl-S-iso-propyl-, O-Ethyl-S-methyl-, O-Ethyl-S-n-propyl-, O-Ethyl-S-iso-propyl-, O-n-Propyl-S-methyl-, O-n-Propyl-S-ethyl-, O-n-Propyl-S-iso-propyl-, O-iso-Propyl-S-methyl-, O-iso-Propyl-S-ethyl- und O-iso-Propyl-S-n-propyl(thiono)thiophosphorsäurediesterchlorid;

O-Methyl-N-(di)methyl-, O-Methyl-N-(di)ethyl-, O-Methyl-N-(di)-n-propyl-, O-Methyl-N-(di)-iso-propyl-, O-Ethyl-N-(di)methyl-, O-Ethyl-N-(di)ethyl-, O-Ethyl-N-(di)-n-propyl-, O-Ethyl-N-(di)-iso-propyl-, O-n-Propyl-N-(di)-methyl-, O-n-Propyl-N-(di)ethyl-, O-n-Propyl-N-(di)-n-propyl-, O-n-Propyl-N-(di)-iso-propyl-, O-iso-Propyl-N-(di)methyl-, O-iso-Propyl-N-(di)ethyl-, O-iso-Propyl-N-(di)-n-propyl(thiono)phosphorsäureamidesterchlorid;

O-Methyl-, O-Ethyl-, O-n-Propyl-, O-iso-Propyl-, O-n-Butyl-, O-iso-Butyl-, O-sek.-Butyl(thiono)phenylphosphonsäureesterchlorid.

Die Verbindungen der Formel III sind bekannt oder können nach allgemein üblichen bekannten Verfahren hergestellt werden (vgl. „Methoden der organischen Chemie", Houben-Weyl-Müller, 4. Aufl., Bd. 12/1 [1963], S. 560-563; Bd. 12/2 [1964], S. 607-618, Thieme-Verlag, Stuttgart).

Das erfindungsgemässe Verfahren wird vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methyl-iso-propyl- und Methyl-iso-butylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines grösseren Bereichs variiert werden. Im allgemeinen arbeitet man zwischen 0 und 150, vorzugsweise zwischen 10 und 100°C.

Das erfindungsgemässe Verfahren wird im allgemeinen bei Normaldruck durchgeführt.

Zur Durchführung des erfindungsgemässen Verfahrens werden die Ausgangsstoffe gewöhnlich in angenähert äquimolaren Mengen eingesetzt. Ein grösserer Überschuss der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt.

Die Isolierung der Verbindungen der Formel I erfolgt in üblicher Weise. Zur Charakterisierung der Verbindungen der Formel I dient der Schmelzpunkt bzw. der Brechungsindex.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der *Isopoda* z.B. *Oniscus asellus, Armadillidium vulgare, Porcellio scaber.*

Aus der Ordnung der *Diplopoda* z.B. *Blaniulus guttulatus.*

Aus der Ordnung der *Chilopoda* z.B. *Geophilus carpophagus, Scutigera spec.*

Aus der Ordnung der *Symphyla* z.B. *Scutigerella immaculata.*

Aus der Ordnung der *Thysanura* z.B. *Lepisma saccharina.*

Aus der Ordnung der *Collembola* z.B. *Onychiurus armatus.*

Aus der Ordnung der *Orthoptera* z.B. *Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.*

Aus der Ordnung der *Dermaptera* z.B. *Forficula auricularia.*

Aus der Ordnung der *Isoptera* z.B. *Reticulitermes spp.*

Aus der Ordnung der *Anoplura* z.B. *Phylloxera vastatrix, Pemphigus spp., Pediculus humanus corporis, Haematopinus spp., Linognathus spp.*

Aus der Ordnung der *Mallophaga* z.B. *Trichodectes spp., Damalinea spp.*

Aus der Ordnung der *Thysanoptera* z.B. *Hercinothrips femoralis, Thrips tabaci.*

Aus der Ordnung der *Heteroptera* z.B. *Eurygaster spp., Dysdercus intermedius, Piesma quadrata, Cimex lectularius, Rhodnius prolixus, Triatoma spp.*

Aus der Ordnung der *Homoptera* z.B. *Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.*

Aus der Ordnung der *Lepidoptera* z.B. *Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.*

Aus der Ordnung der *Coleoptera* z.B. *Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus*

*spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.*

Aus der Ordnung der *Hymenoptera* z.B. *Diprion spp., Hoplocampa spp., Lasius spp., Monomorium pharaonis, Vespa spp.*

Aus der Ordnung der *Diptera* z.B. *Aedes spp., Anopheles spp., Culex spp., Drosophila melanogaster, Musca spp., Fannia spp., Calliphora erythrocephala, Lucilla spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.*

Aus der Ordnung der *Siphonaptera* z.B. *Xenopsylla cheopis, Ceratophyllus spp.*

Aus der Ordnung der *Arachnida* z.B. *Scorpio maurus, Latrodectus mactans.*

Aus der Ordnung der *Acarina* z.B. *Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.*

Zu den pflanzenparasitären Nematoden gehören *Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.*

Die Wirkstoffe können in die üblichen Formulierungen übergeführt werden, wie Lösungen, Emulsionen, Suspensionen, Pulver, Schäume, Pasten, Granulate, Aerosole, Wirtstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen, sowie ULV-Kalt- und -Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylenfettsäureester, Polyoxyethylenenfettalkoholether, z.B. Alkylarylpolyglykolether, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 Gew.-%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-%, liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirksamkeit der erfindungsgemässen Verbindungen sei anhand der folgenden Biespiele erläutert, wobei teilweise mit den aus dem eingangs erwähnten Stand der Technik bekannten Vergleichsverbindungen A und/oder B vergleichend geprüft wurde.

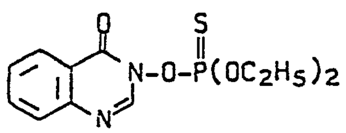

Vergleichsverbindung A

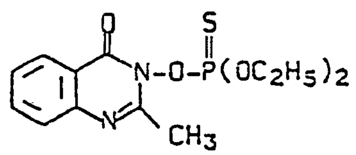

Vergleichsverbindung B

*Beispiel A:*

*Plutella*-Test

Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

Kohlblätter *(Brassica oleracea)* werden durch Tauchen in die Wirkstoffzubereitung der gewünschten Konzentration behandelt und mit Raupen der Kohlschabe *(Plutella maculipennis)* besetzt, solange die Blätter noch feucht sind.

Nach der gewünschten Zeit wird die Abtötung in Prozent bestimmt. Dabei bedeutet 100%, dass alle Raupen abgetötet wurden; 0% bedeutet, dass keine Raupen abgetötet wurden.

Bei diesem Test zeigten z.B. bei einer Konzentration von 0,001% nach 3 d die Verbindungen der Herstellungsbeispiele 1, 4 und 5 eine Abtötung von 90 bis 100%, während die Vergleichsverbindung A eine Abtötung von 30% ergab.

*Beispiel B:*

*Myzus*-Test

Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

Kohlblätter *(Brassica oleracea)*, die stark von der Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in Prozent bestimmt. Dabei bedeutet 100%, dass alle Blattläuse abgetötet wurden; 0% bedeutet, dass keine Blattläuse abgetötet wurden.

Bei diesem Test zeigten z.B. bei einer Konzentration von 0,1% nach 1 d die Verbindungen der Herstellungsbeispiele 1, 2, 4 und 5 eine Abtötung von 95 bis 100%, während die Vergleichsverbindung B eine Abtötung von 60% ergab.

*Beispiel C:*

*Tetranychus*-Test (resistent)

Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

Bohnenpflanzen *(Phaseolus vulgaris)*, die stark von allen Entwicklungsstadien der gemeinen Spinnmilbe oder Bohnenspinnmilbe *(Tetranychus urticae)* befallen sind, werden durch Tauchen in die Wirkstoffzubereitung der gewünschten Konzentration behandelt.

Nach der gewünschten Zeit wird die Abtötung in Prozent bestimmt. Dabei bedeutet 100%, dass alle Spinnmilben abgetötet wurden; 0% bedeutet, dass keine Spinnmilben abgetötet wurden.

Bei diesem Test zeigten z.B. bei einer Wirkstoffkonzentration von 0,1% nach 2 d die Verbindungen der Herstellungsbeispiele 1 und 2 eine Abtötung von 70 bis 90%, während die Vergleichsverbindung B keine Abtötung (0%) ergab.

*Beispiel D:*

Grenzkonzentrations-Test/Bodeninsekten

Testinsekt: *Phorbia antiqua*-Maden im Boden
Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche

in ppm (= mg/l) angegeben wird. Man füllt den Boden in Töpfe und lässt diese bei Raumtemperatur stehen.

Nach 24 h werden die Testtiere in den behandelten Boden gegeben und nach weiteren 2 bis 7 d wird der Wirkungsgrad des Wirkstoffs durch Auszählen der toten und lebenden Testinsekten in Prozent bestimmt. Der Wirkungsgrad ist 100%, wenn alle Testinsekten abgetötet worden sind, er ist 0%, wenn noch genauso viele Testinsekten leben wie bei der unbehandelten Kontrolle.

Bei diesem Test zeigten z.B. bei einer Konzentration von 10 ppm die Verbindungen der Herstellungsbeispiele 1, 3 und 4 eine Abtötung von 100%, während Vergleichsverbindung B keine Abtötung (0%) ergab.

*Beispiel E:*

Grenzkonzentrations-Test/Bodeninsekten

Testinsekt: *Tenebrio molitor*-Larven im Boden
Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 h werden die Testtiere in den behandelten Boden gegeben und nach weiteren 2 bis 7 d wird der Wirkungsgrad des Wirkstoffs durch Auszählen der toten und lebenden Testinsekten in Prozent bestimmt. Der Wirkungsgrad ist 100%, wenn alle Testinsekten abgetötet worden sind, er ist 0%, wenn noch genauso viele Testinsekten leben wie bei der unbehandelten Kontrolle.

Bei diesem Test zeigten z.B. bei einer Konzentration von 20 ppm die Verbindungen der Herstellungsbeispiele 1, 2, 4 und 5 eine Abtötung von 100%, während die Vergleichsverbindungen A und B keine Abtötung (0%) ergaben.

Das erfindungsgemässe Verfahren soll durch die folgenden Herstellungsbeispiele erläutert werden.

*Beispiel 1:*

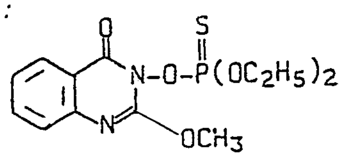

Eine Mischung aus 6,8 g (0,035 mol) 3,4-Dihydro-3-hydroxy-2-methoxy-4-oxochinazolin, 7,2 g (0,052 mol) Kaliumcarbonat, 6,6 g (0,035 mol) O,O-Diethylthionophosphorsäurediesterchlorid und 100 ml Acetonitril wird 1 h bei 45-50°C gerührt. Dann gibt man 200 ml Toluol zu, schüttelt zweimal mit je 200 ml Wasser und trocknet die organische Phase über Natriumsulfat. Nach Abdestillieren des Lösungsmittels erhält man 9,2 g (76% der Theorie) O,O-Diethyl-O-3,4-dihydro-2-methoxy-4-oxochinazolin-3-ylthionophosphorsäureester in Form farbloser Kristalle mit dem Schmelzpunkt 74°C.

Analog Beispiel 1 können die folgenden Verbindungen der Formel I

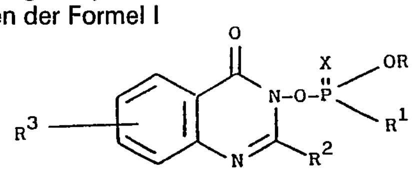 (I)

hergestellt werden.

| Beispiel Nr. | R | $R^1$ | $R^2$ | $R^3$ | X | Ausbeute (% d. T.) | Brechungsindex; Schmelzpunkt (°C) |
|---|---|---|---|---|---|---|---|
| 2 | $C_3H_7$-iso | $CH_3$ | $OCH_3$ | H | S | 80 | 97 |
| 3 | $C_2H_5$ | $NH-C_3H_7$-iso | $OCH_3$ | H | S | 80 | 108 |
| 4 | $C_2H_5$ | $OC_2H_5$ | $OC_2H_5$ | H | S | 77 | 67 |
| 5 | $C_2H_5$ | $OC_2H_5$ | $OCH_3$ | 7-Cl | S | 84 | 67 |
| 6 | $CH_3$ | $OCH_3$ | $OCH_3$ | H | S | | 69 |
| 7 | $C_2H_5$ | $SC_3H_7$-n | $OCH_3$ | H | S | | $n_D^{22} = 1{,}5773$ |
| 8 | $C_2H_5$ | $SC_3H_7$-n | $OCH_3$ | H | O | | |
| 9 | $C_2H_5$ | 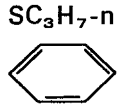 | $OCH_3$ | H | S | | |
| 10 | $C_2H_5$ | $OC_2H_5$ | $OCH_3$ | H | O | | |
| 11 | $C_2H_5$ | $OC_2H_5$ | $OC_3H_7$-n | H | S | | |
| 12 | $C_2H_5$ | $OC_2H_5$ | $OC_3H_7$-iso | H | S | | |
| 13 | $CH_3$ | $OCH_3$ | $SCH_3$ | H | S | | |
| 14 | $CH_3$ | $OCH_3$ | $N(CH_3)_2$ | H | S | | |
| 15 | $C_2H_5$ | $OC_2H_5$ | $N(CH_3)_2$ | H | S | | |
| 16 | $C_2H_5$ | $OC_2H_5$ | $SCH_3$ | H | S | | |
| 17 | $C_2H_5$ | $OC_2H_5$ | $OC_4H_9$-sek. | H | S | | |
| 18 | $CH_3$ | $OCH_3$ | $SC_2H_5$ | H | S | | |

| Beispiel Nr. | R | R¹ | R² | R³ | X | Ausbeute (% d. T.) | Brechungsindex; Schmelzpunkt (°C) |
|---|---|---|---|---|---|---|---|
| 19 | $C_2H_5$ | $OC_2H_5$ | $SC_2H_5$ | H | S | | |
| 20 | $C_2H_5$ | $OC_2H_5$ | $SC_3H_7$-iso | H | S | | |
| 21 | $C_2H_5$ | $OC_2H_5$ | $N(C_2H_5)_2$ | H | S | | |
| 22 | $-CH_3$ | $-OC_3H_7$-n | $-OCH_3$ | H | S | 87 | $n_D^{19} = 1,5525$ |
| 23 | $-C_2H_5$ | $-OC_3H_7$-n | $-OCH_3$ | H | S | 91 | $n_D^{19} = 1,5441$ |
| 24 | $-C_3H_7$-n | $-OC_3H_7$-n | $-OCH_3$ | H | S | 87 | $n_D^{19} = 1,5439$ |
| 25 | $-C_2H_5$ | $-OCH_2CH_2-OCH_3$ | $-OCH_3$ | H | S | 87 | $n_D^{19} = 1,5538$ |
| 26 | $-C_2H_5$ | $-OC_4H_9$-n | $-OCH_3$ | H | S | | $n_D^{18} = 1,5388$ |

Die als Vorprodukte zu verwendenden 3,4-Dihydro-3-hydroxy-4-oxochinazoline der Formel II können z.B. wie folgt hergestellt werden.

*Beispiel a:*

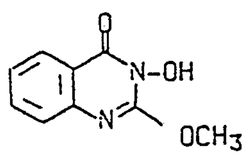

Eine Mischung aus 22,8 g (0,15 mol) O-Aminobenzhydroxamsäure, 50 ml Methanol und 27,2 g (0,2 mol) Orthokohlensäuretetramethylester wird 2 h unter Rückfluss gekocht und dann auf 5°C abgekühlt. Das auskristallisierte Produkt wird abgesaugt. Man erhält so 17 g (60% der Theorie) 2-Methoxy-3-hydroxy-3,4-dihydro-4-oxochinazolin in Form farbloser Kristalle mit dem Schmelzpunkt 220°C.

Analog diesem Beispiel können die folgenden Verbindungen der Formel II

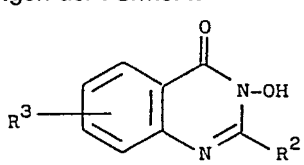

(II)

hergestellt werden.

| Beispiel Nr. | R² | R³ | Ausbeute (% d. T.) | Schmelzpunkt (°C) |
|---|---|---|---|---|
| b | $OC_2H_5$ | H | 86 | 154 |
| c | $OCH_3$ | 7-Cl | 44 | 206 |
| d | $OC_3H_7$-n | | | |
| e | $OC_3H_7$-iso | | | |
| f | $OC_4H_9$-sek. | | | |

**Patentansprüche**

1. Oxochinazolin(thiono)phosphor(phosphon)säureester bzw. -esteramide der Formel (I)

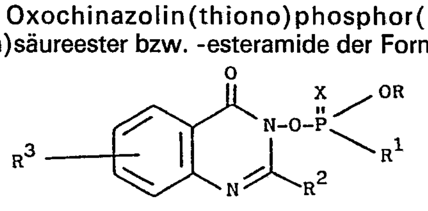

(I)

in welcher

R für gegebenenfalls substituiertes Alkyl steht,

R¹ für einen gegebenenfalls substituierten Alkyl-, Alkoxy-, Alkylthio-, Alkylamino-, Dialkylamino- oder Arylrest steht,

R² für einen gegebenenfalls substituierten Alkoxy-, Alkylthio-, Alkylamino- oder Dialkylaminorest steht,

R³ für Wasserstoff oder Halogen steht, und

X für Sauerstoff oder Schwefel steht.

2. Verbindungen der Formel (I) gemäss Anspruch 1, in welcher

R für gegebenenfalls substituiertes Alkyl mit 1 bis 6 Kohlenstoffatomen steht,

R¹ für einen gegebenenfalls substituierten Alkyl-, Alkoxy-, Alkylthio-, Alkylamino- oder Dialkylaminorest mit jeweils 1 bis 6 Kohlenstoffatomen je Alkylteil oder Phenyl steht,

R² für einen gegebenenfalls substituierten Alkoxy-, Alkylthio-, Alkylamino- oder Dialkylaminorest mit jeweils 1 bis 6 Kohlenstoffatomen je Alkylteil steht,

R³ für Wasserstoff, Fluor, Chlor oder Brom steht, und

X für Sauerstoff oder Schwefel steht.

3. Verbindungen der Formel (I) gemäss Anspruch 1, in welcher

R für Alkyl mit 1 bis 6 Kohlenstoffatomen steht,

R¹ für Alkyl, Alkoxy, Alkoxyalkoxy, Alkylthio, Alkylamino oder Dialkylamino mit jeweils 1 bis 6 Kohlenstoffatomen je Alkylteil oder Phenyl steht,

R² für Alkoxy, Alkylthio, Alkylamino oder Dialkylamino mit jeweils 1 bis 6 Kohlenstoffatomen je Alkylteil steht,

R³ für Wasserstoff, Fluor, Chlor oder Brom steht, und

X für Sauerstoff oder Schwefel steht.

4. Verbindung der Formel

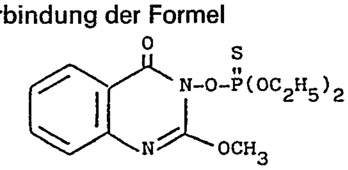

5. Verfahren zur Herstellung von Oxochinazolin(thiono)phosphor(phosphon)säureestern bzw. -esteramiden der Formel (I)

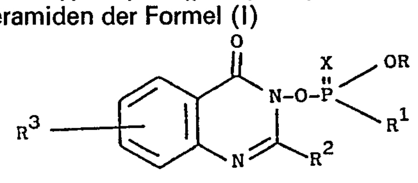

(I)

in welcher

R für gegebenenfalls substituiertes Alkyl steht,

$R^1$ für einen gegebenenfalls substituierten Alkyl-, Alkoxy-, Alkylthio-, Alkylamino-, Dialkylamino- oder Arylrest steht,

$R^2$ für einen gegebenenfalls substituierten Alkoxy-, Alkylthio-, Alkylamino- oder Dialkylaminorest steht,

$R^3$ für Wasserstoff oder Halogen steht, und

X für Sauerstoff oder Schwefel steht,

dadurch gekennzeichnet, dass man 3,4-Dihydro-3-hydroxy-4-oxochinazoline der Formel (II)

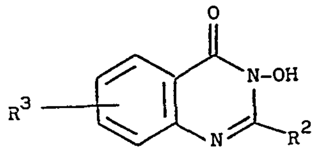
(II)

in welcher

$R^2$ und $R^3$ die oben angegebene Bedeutung haben,

mit (Thiono)Phosphor(phosphon)säureester- bzw. -esteramidhalogeniden der Formel (III)

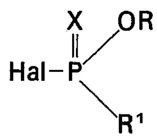
(III)

in welcher

R, $R^1$ und X die oben angegebene Bedeutung haben, und

Hal für Halogen steht,

gegebenenfalls in Gegenwart von Säureakzeptoren und gegebenenfalls in Gegenwart von Verdünnungsmitteln umsetzt.

6. Schädlingsbekämpfungsmittel, gekennzeichnet durch einen Gehalt an mindestens einer Verbindung der Formel (I).

7. Verwendung von Verbindungen der Formel (I) zur Bekämpfung von Schädlingen, insbesondere Insekten und Spinnentieren.

8. Verfahren zur Bekämpfung von Schädlingen, dadurch gekennzeichnet, dass man Verbindungen der Formel (I) auf die Schädlinge, vorzugsweise Insekten oder Spinnentiere oder ihren Lebensraum einwirken lässt.

9. Verfahren zur Herstellung von Schädlingsbekämpfungsmitteln, dadurch gekennzeichnet, dass man Verbindungen der Formel (I) mit Streckmitteln und/oder oberflächenaktiven Mitteln vermischt.

10. 3,4-Dihydro-3-hydroxy-4-oxochinazoline der Formel (II)

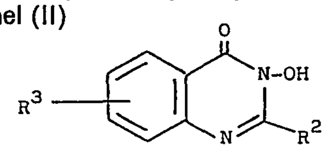
(II)

in welcher

$R^2$ und $R^3$ die in den Ansprüchen 1 bis 3 angegebene Bedeutung haben.

**Claims**

1. Oxo-quinazoline-(thiono)-phosphoric-(phosphonic) acid esters and ester amides of the Formula (I)

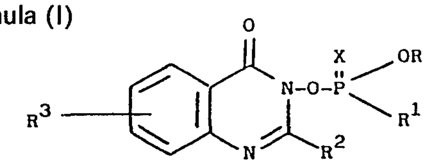
(I)

in which

R represents optionally substituted alkyl,

$R^1$ denotes an optionally substituted alkyl, alkoxy, alkylthio, alkylamino, dialkylamino or aryl radical,

$R^2$ represents an optionally substituted alkoxy, alkylthio, alkylamino or dialkylamino radical,

$R^3$ represents hydrogen or halogen, and

X represents oxygen or sulphur.

2. Compounds of the Formula (I) according to Claim 1,

in which

R represents optionally substituted alkyl having 1 to 6 carbon atoms,

$R^1$ represents an optionally substituted alkyl, alkoxy, alkylthio, alkylamino or dialkylamino radical having in each case 1 to 6 carbon atoms per alkyl part or phenyl,

$R^2$ represents an optionally substituted alkoxy, alkylthio, alkylamino or dialkylamino radical having in each case 1 to 6 carbon atoms per alkyl part,

$R^3$ represents hydrogen, fluorine, chlorine or bromine, and

X represents oxygen or sulphur.

3. Compounds of the Formula (I) according to Claim 1,

in which

R represents alkyl having 1 to 6 carbon atoms,

$R^1$ represents alkyl, alkoxy, alkoxyalkoxy, alkylthio, alkylamino or dialkylamino having in each case 1 to 6 carbon atoms per alkyl part or phenyl,

$R^2$ represents alkoxy, alkylthio, alkylamino or dialkylamino having in each case 1 to 6 carbon atoms per alkyl part,

$R^3$ represents hydrogen, fluorine, chlorine or bromine, and

X represents oxygen or sulphur.

4. Compound of the formula

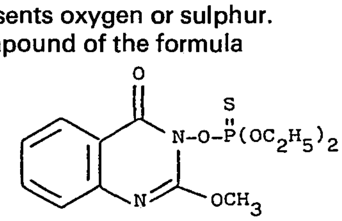

5. Process for the preparation of oxo-quinazoline-(thiono)-phosphoric(phosphonic) acid esters and ester amides of the Formula (I)

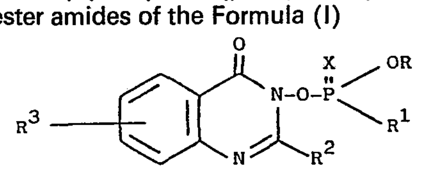
(I)

in which

R represents optionally substituted alkyl,

$R^1$ denotes an optionally substituted alkyl, alkoxy, alkylthio, alkylamino, dialkylamino or aryl radical,

$R^2$ represents an optionally substituted alkoxy, alkylthio, alkylamino or dialkylamino radical,

$R^3$ represents hydrogen or halogen, and

X represents oxygen or sulphur,

characterised in that 3,4-dihydro-3-hydroxy-4-oxo-quinazolines of the Formula (II)

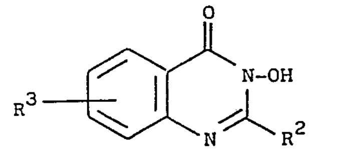
(II)

in which $R^2$ and $R^3$ have the above-mentioned meaning, are reacted with (thiono)-phosphoric-(phosphonic) acid ester halides or ester amide halides of the Formula (III)

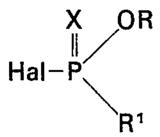
(III)

in which R, $R^1$ and X have the above-mentioned meaning and Hal represents halogen, if appropriate in the presence of acid acceptors and if appropriate in the presence of diluents.

6. Pest-combating agents, characterised in that they contain at least one compound of the Formula (I).

7. Use of compounds of the Formula (I) for combating pests, in particular insects and arachnidae.

8. Process for combating pests, characterised in that compounds of the Formula (I) are allowed to act on the pests, preferably insects or arachnidae, or their habitat.

9. Process for the preparation of pest-combating agents, characterised in that compounds of the Formula (I) are mixed with extenders and/or surface-active agents.

10. 3,4-Dihydro-3-hydroxy-4-oxo-quinazolines of the Formula (II)

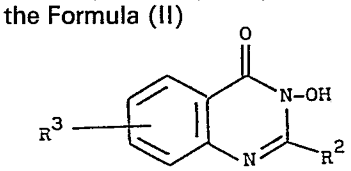
(II)

in which $R^2$ and $R^3$ have the meaning indicated in Claims 1 to 3.

## Revendications

1. Esters ou estéramides d'acides oxoquinazoline(thiono)phosphoro(phosphoniques) de formule (I)

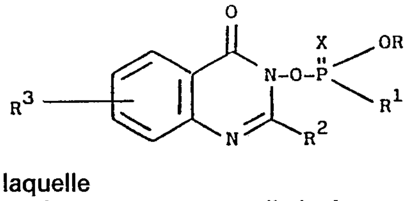
(I)

dans laquelle

R représente un groupe alkyle éventuellement substitué,

$R^1$ représente un groupe aryle, dialkylamino, alkylamino, alkylthio, alcoxy ou alkyle éventuellement substitué,

$R^2$ représente un groupe dialkylamino, alkylamino, alkylthio ou alcoxy éventuellement substitué.

$R^3$ représente un atome d'hydrogène ou d'halogène, et

X représente un atome d'oxygène ou de soufre.

2. Composés de formule (I) suivant la revendication 1,

dans laquelle

R représente un groupe alkyle éventuellement substitué et contenant 1 à 6 atomes de carbone,

$R^1$ représente un groupe dialkylamino, alkylamino, alkylthio, alcoxy ou alkyle éventuellement substitué contenant 1 à 6 atomes de carbone dans chaque fraction alkyle, ou un groupe phényle,

$R^2$ représente un groupe dialkylamino, alkylamino, alkylthio ou alcoxy éventuellement substitué contenant 1 à 6 atomes de carbone dans chaque fraction alkyle,

$R^3$ représente un atome d'hydrogène, de fluor, de chlore ou de brome, et

X représente un atome d'oxygène ou de soufre.

3. Composés de formule (I) suivant la revendication 1,

dans laquelle

R représente un groupe alkyle contenant 1 à 6 atomes de carbone,

$R^1$ représente un groupe dialkylamino, alkylamino, alkylthio, alcoxyalcoxy, alcoxy ou alkyle contenant 1 à 6 atomes de carbone dans chaque fraction alkyle, ou un groupe phényle,

$R^2$ représente un groupe dialkylamino, alkylamino, alkylthio ou alcoxy contenant 1 à 6 atomes de carbone dans chaque fraction alkyle,

$R^3$ représente un atome d'hydrogène, de fluor, de chlore ou de brome, et

X représente un atome d'oxygène ou de soufre.

4. Composé de formule

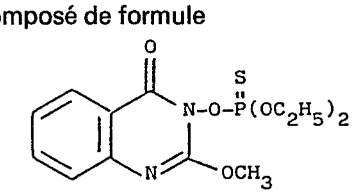

5. Procédé de préparation d'esters ou d'estéramides d'acides oxoquinazoline(thiono)phosphoro(phosphoniques) de formule (I)

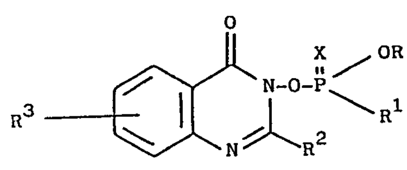
(I)

dans laquelle

R représente un groupe alkyle éventuellement substitué,

$R^1$ représente un groupe aryle, dialkylamino, alkylamino, alkylthio, alcoxy ou alkyle éventuellement substitué,

$R^2$ représente un groupe dialkylamino, alkylamino, alkylthio ou alcoxy éventuellement substitué,

$R^3$ représente un atome d'hydrogène ou d'halogène, et

X représente un atome d'oxygène ou de soufre, caractérisé en ce qu'on fait réagir des 3,4-dihydro-3-hydroxy-4-oxoquinazolines de formule (II)

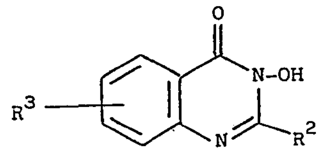

(II)

dans laquelle $R^2$ et $R^3$ ont les significations indiquées ci-dessus, avec des halogénures d'esters ou d'estéramides d'acides (thiono)phosphoro(phosphoniques) de formule (III)

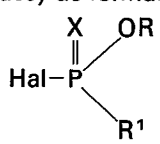

(III)

dans laquelle R, $R^1$ et X ont les significations indiquées ci-dessus, et Hal représente un atome d'halogène, éventuellement en présence d'accepteurs d'acides et éventuellement en présence de diluants.

6. Parasiticides, caractérisés en ce qu'ils contiennent au moins un composé de formule (I).

7. Utilisation de composés de formule (I) pour combattre des parasites, en particulier des insectes et des acariens.

8. Procédé en vue de combattre des parasites, caractérisé en ce qu'on fait agir des composés de formule (I) sur les parasites, de préférence les insectes ou les acariens, ou encore sur leur biotope.

9. Procédé de préparation de parasiticides, caractérisé en ce qu'on mélange des composés de formule (I) avec des diluants et/ou des agents tensio-actifs.

10. 3,4-dihydro-3-hydroxy-4-oxoquinazolines de formule (II)

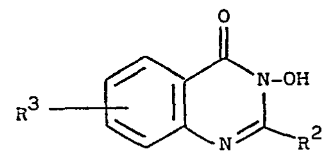

(II)

dans laquelle $R^2$ et $R^3$ ont les significations indiquées dans les revendications 1 à 3.